(19) Europäisches Patentamt / European Patent Office / Office européen des brevets

(11) **EP 3 859 010 A1**

(12) **EUROPEAN PATENT APPLICATION**
published in accordance with Art. 153(4) EPC

(43) Date of publication:
04.08.2021  Bulletin 2021/31

(51) Int Cl.:
*C12Q 1/68* (2018.01)  *C12N 15/11* (2006.01)
*G16B 30/00* (2019.01)  *G16H 50/30* (2018.01)

(21) Application number: 19867890.6

(22) Date of filing: 29.09.2019

(86) International application number:
**PCT/CN2019/109036**

(87) International publication number:
**WO 2020/063964 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  29.09.2018  CN 201811149015
29.09.2018  CN 201811149011

(71) Applicant: **Guangzhou Burning Rock DX Co., Ltd.**
**Guangzhou, Guangdong 510300 (CN)**

(72) Inventors:
• **HAN, Yusheng**
**Guangzhou, Guangdong 510300 (CN)**
• **LIU, Chenglin**
**Guangzhou, Guangdong 510300 (CN)**
• **ZHANG, Zhihong**
**Guangzhou, Guangdong 510300 (CN)**
• **ZHANG, Zhou**
**Guangzhou, Guangdong 510300 (CN)**
• **DUAN, Feidie**
**Guangzhou, Guangdong 510300 (CN)**

(74) Representative: **Zacco GmbH**
**Bayerstrasse 83**
**80335 München (DE)**

(54) **SECOND GENERATION SEQUENCING-BASED METHOD FOR DETECTING MICROSATELLITE STABILITY AND GENOME CHANGES BY MEANS OF PLASMA**

(57)   In one aspect, the present disclosure relates to a panel of biomarkers, a kit for detecting it, and its use in the detection of micro satellite instability (MSI) as well as non-invasive diagnosis, prognostic evaluation, selection of treatment or genetic screening of cancer, preferably colorectal cancer (such as bowel cancer), gastric cancer or endometrial cancer in a plasma sample. On the other hand, the present disclosure provides a method for detecting microsatellite instability (MSI) and disease-related gene mutations through plasma based on next-generation sequencing, and a device for implementing the method, especially the use of such detection method in the non-invasive diagnosis, prognostic evaluation, selection of treatment or genetic screening of patients with cancer, preferably colorectal cancer (such as bowel cancer), gastric cancer or endometrial cancer. This disclosure provides a plasma MSI detection method for the first time, which can determine the microsatellite instability of a sample with high accuracy and sensitivity.

EP 3 859 010 A1

**Description**

This disclosure claims the priority of the application filed on September 29, 2018, with the application number of 201811149011.0, and titled "Next-generation sequencing-based method for detection of microsatellites stability and genomic changes through plasma detection" and the application filed on September 29, 2018, with the application number of 201811149015.9, and titled "Microsatellite biomarker panel, detection kit and use thereof".

**Field of the Invention**

The present disclosure relates to a biomarker panel, a kit for detecting it, a method for detection of microsatellite stability in a plasma sample with it, and its use in non-invasive diagnosis, prognostic evaluation, selection of treatment or genetic screening of cancer, preferably colorectal cancer (such as bowel cancer), gastric cancer or endometrial cancer.

**Background of the Invention**

A microsatellite is a repetitive DNA short sequence or single nucleotide region within the genome. In tumor cells, when DNA methylation or gene mutations cause the disfunction of mismatch repair genes, microsatellite repetitive sequence mismatch (microsatellite mutation) can be caused, leading to its sequence being shortened or lengthened, thereby resulting in microsatellite instability (MSI). According to the degree of MSI, it can be classified into types of microsatellite instability-high (MSI-H), microsatellite instability-low (MSI-L), and microsatellite stable (MSS).

A large number of studies have shown that MSI is involved in the development of malignant tumors and is closely related to colorectal cancer (such as bowel cancer), gastric cancer and endometrial cancer. As an example, there is MSI-H phenotype among about 15% of patients with colorectal cancer, and among more than 90% of patients with typical hereditary nonpolyposis colorectal cancer (HNPCC) therein, indicating that MSI-H can be used as an important marker for detecting whether the patients have HNPCC. Patients with MSI-H colorectal cancer have a better prognosis, compared with those with MSS (i.e. microsatellite stable) colorectal cancer. Their drug responses are different, suggesting that MSI-H can be used as an independent predictor of colorectal cancer prognosis. Therefore, MSI detection is of great significance for patients with colorectal cancer.

The latest edition of the 2016 year's National Comprehensive Cancer Network (NCCN, 2016 Version 2) guidelines for colorectal cancer treatment clearly states for the first time that "all patients with a history of colon/rectal cancer should be tested for MMR (mismatch repair) or MSI", because the prognosis for MSI-H (i.e., high microsatellite instability) stage II colorectal cancer patients is good (5y-OS rate for surgery alone is 80%) and the patients cannot benefit from 5FU adjuvant chemotherapy (which is however harmful). And the guidelines recommend for the first time PD-1 monoclonal antibody Pembrolizumab and Nivolumab for the end-line therapy of the mCRC's patients with dMMR/MSI-H molecular phenotype. This fully demonstrates the importance of detecting MMR and MSI in advanced colorectal cancer. At the same time, due to the association of a large number of genes with hereditary colorectal cancer, it is recommended for the patients and their families with a clear family history to employ multi-gene panel sequencing for the first detection.

In 2017, Merck's PD-1 monoclonal antibody Keytruda was approved by the FDA in USA for the treatment of solid tumor patients with MSI-H or mismatch repair defects (dMMR), which once again proved that MSI-H can be used as a pan-cancer tumor marker independent of tumor location. Therefore, MSI detection of cancer is very important.

At present, MSI detection methods are limited to detection of tissues. For example, MMR genetic detection carried out in domestic hospitals usually detects MLH1 and MSH2 only, and some of them also detects both MSH6 and PMS2, and the positive results thereof is less consistent with the MSI detection results. Only a few hospitals have carried out MSI state detection by PCR combined with capillary electrophoresis method, and most of them are outsource detection. This method usually selects 5-11 single nucleotide repeat sites with a length of about 25 bp. After PCR operation, the length distribution interval is measured by capillary electrophoresis to determine the microsatellite instability of the sample. This method is the current gold standard detection method. Recently, the method for detection of MSI in tissues based on next-generation sequencing has been proved to have an extremely high coincidence rate with PCR-MSI, which can depict the genome map while judging the MSI status, and provide more information for cancer diagnosis. However, all of these methods require a sufficient proportion of tumor cells. Since circulating tumor DNA (ctDNA) is extremely little in plasma, tissue-based methods cannot be implemented in plasma.

Tumor blood detection has the characteristics of non-invasive, real-time, and non-tissue specificity that tissues do not have, and has important clinical significance. Therefore, there is an urgent need in the art for plasma-based MSI detection methods, especially for the method for detection of MSI in tumor blood in non-invasive diagnosis, prognosis evaluation, selection of treatment or genetic screening for cancer, preferably colorectal cancer (such as bowel cancer), gastric cancer or endometrial cancer.

**Summary of the Invention**

This disclosure provides a method for detection of MSI in plasma for the first time, and compared with MSI detection in tissues, the plasma MSI detection of this disclosure is non-invasive, real-time, non-tissue specific, and can detect multiple lesions in advance. At the same time, the method of the present disclosure can complete the detection of microsatellite status in plasma samples with very low ctDNA content, filling the gap in the detection of microsatellite status through plasma samples. It has fast detection speed, does not rely on matching white blood cell samples, has lower prices, has faster detection and can determine the microsatellite stable (MS) status of the sample with high accuracy, high sensitivity and high specificity.

At the same time, the detection method of the present disclosure can also be used for non-invasive diagnosis, prognostic evaluation, or selection of treatment for patients with colorectal cancer (such as bowel cancer), gastric cancer or endometrial cancer.

Specifically, this disclosure relates to the following aspects:
In one aspect, the present disclosure provides a biomarker panel comprising one or more of 8 microsatellite loci as shown in Table 1.

In another aspect, the present disclosure provides a biomarker panel comprising a combination of microsatellite loci and one or more genes, wherein the microsatellite loci comprise the 8 microsatellite loci shown in claim 1, or any one of them, or a combination of some of them, wherein the one or more genes are any one or more of the following 41 genes: AKT1, APC, ATM, BLM, BMPR1A, BRAF, BRCA1, BRCA2, CDH1, CHEK2, CYP2D6, DPYD, EGFR, EPCAM, ERBB2, GALNT12, GREM1, HRAS, KIT, KRAS, MET, MLH1, MSH2, MSH6, MUTYH, NRAS, PDGFRA, PIK3CA, PMS1, PMS2, POLD1, POLE, PTCH1, PTEN, SDHB, SDHC, SDHD, SMAD4, STK11, TP53, UGT1A1.

In another aspect, the present disclosure provides a kit for the detection of microsatellite stability in a plasma sample, characterized in that the kit comprises a detection reagent for the biomarker panel used in the present disclosure.

In yet another aspect, the present disclosure provides a kit for use in the non-invasive diagnosis, prognostic evaluation, selection of treatment or genetic screening of cancer, preferably colorectal cancer (such as bowel cancer), gastric cancer or endometrial cancer, characterized in that the kit comprises a detection reagent for the biomarker panel used in the present disclosure.

Preferably, in the kit provided by the present disclosure, the plasma sample is a cancer plasma sample, preferably a colorectal cancer plasma sample, such as a bowel cancer plasma sample, a gastric cancer plasma sample, and an endometrial cancer plasma sample.

More preferably, the microsatellite stability comprises types of microsatellite instability-high (MSI-H), microsatellite instability-low (MSI-L), and microsatellite stable (MSS).

In one embodiment, in the kit provided by the present disclosure, the detection reagent is a reagent for performing high-throughput next-generation sequencing (NGS).

Additionally, the present disclosure further relates to use of the biomarker panel in detection of the microsatellite stability in a plasma sample.

Preferably, the plasma sample is a cancer plasma sample, preferably a colorectal cancer plasma sample, such as a bowel cancer plasma sample, a gastric cancer plasma sample, and an endometrial cancer plasma sample.

More preferably, the microsatellite stability comprises types of microsatellite instability-high (MSI-H), microsatellite instability-low (MSI-L), and microsatellite stable (MSS).

Additionally, the present disclosure further relates to use of the biomarker panel in the non-invasive diagnosis, prognostic evaluation, selection of treatment or genetic screening of cancer, preferably colorectal cancer (such as bowel cancer), gastric cancer or endometrial cancer.

In one aspect, the present disclosure provides a method for determining microsatellite marker loci that can be used in the detection of microsatellite stability status in a plasma sample, which comprises the following steps:

1) detecting the microsatellite loci in the sequencing region of the sample;
2) counting the number of reads) of each length types of different repetitive sequence counted by NGS data statistics for any one of the microsatellite loci *i*;
3) determining the length characteristics of the locus repetitive sequence under microsatellite stable (MSS) and the length characteristics of the locus repetitive sequence under microsatellite instability-high (MSI-H) for any one of the microsatellite loci; wherein the length characteristics of MSS is a minimum range of continuous length, such that the number of corresponding reads in the MSS sample is greater than 75% of the total number of reads supported by the locus; the length characteristics of MSI-H is a range of continuous length that is highly differentiated in MSS and MSI-H samples, such that a) the total number of reads supported by this range is less than 0.2% of the total number of reads at the locus in the MSS sample, and b) accounts for more than 50% of the total number of reads at the locus in the MSI-H sample,

the microsatellite locus with the above characteristics being the detection marker of microsatellite locus.

In one embodiment, in the method for determination of microsatellite marker loci, the sample includes a sample from normal white blood cells and tissues from cancer patients, and the cancer is preferably colorectal cancer (such as bowel cancer), gastric cancer or endometrial cancer. Preferably, the microsatellite loci determined using the method for determination of microsatellite marker loci of the present disclosure comprises one or more of the 8 microsatellite loci described in Table 1.

More preferably, in the method for determination of microsatellite marker loci, the detection of microsatellite stability status is used for non-invasive diagnosis, prognostic evaluation, selection of treatment or genetic screening of cancer, preferably colorectal cancer (such as bowel cancer), gastric cancer or endometrial cancer.

In one aspect, the present disclosure provides a method for determining the microsatellite stability loci through a plasma sample of a cancer patient based on the next-generation high-throughput sequencing method, which comprises the following steps:

1) determining the length characteristics of repetitive sequences of multiple microsatellite loci in a plasma sample and an MSS plasma sample as the reference sample based on the next-generation sequencing method, the multiple microsatellite loci comprising one or more of microsatellite loci selected from the 8 microsatellite loci shown in Table 1;
2) calculating its corresponding enrichment index Zscore for any one of microsatellite loci described in 1);
3) summing the enrichment index Zscore of all microsatellite loci to result in an index MSscore for judging the status of microsatellites of the sample;
4) calculating the average value (mean) and standard deviation SD of the MSscore of the MSS plasma sample as the reference sample, with mean + 3SD as the threshold cutoff;
5) determining the sample as MSI-H when MSscore > cutoff and determining the sample as MSS when MSscore ≤ cutoff for a plasma sample from a cancer patient.

In one embodiment, in the method of determining the stability status of microsatellite loci through the plasma samples of cancer patients based on the next-generation high-throughput sequencing method, the Zscore is evaluated by $H_s$,
which is evaluated by $H_s = - \log (P_s(X > k_s))$,
and

$$P(X = k) = \frac{\binom{K}{k}\binom{N-k}{n-k}}{\binom{N}{n}}$$

wherein N is the total number of reads in the repetitive sequence length set for MSI-H status and MSS status, K is the total number of reads in the repetitive sequence length set for MSI-H status, and N - K is the total number of reads in the repetitive sequence length set for MSS status, and correspondingly, $n$ and $k$ are the numbers of respective reads in the sample to be tested, respectively.

In one embodiment, in the method of determining the stability status of microsatellite loci through the plasma samples of cancer patients based on the next-generation high-throughput sequencing method, MSscore is calculated based on the following formula:

$$MSscore = \sum_{s \in markers} \frac{H_s - mean_{MSS\_Sample\ s}(H_s)}{sd_{MSS\_Sample\ s}(H_s)}$$

Preferably, the cancer is colorectal cancer (such as bowel cancer), gastric cancer, or endometrial cancer.

In yet another aspect, the present disclosure provides a method for detecting microsatellite stability status and disease-related gene variations in patients based on next-generation high-throughput sequencing to provide clinical guidance on the risk control, treatment and/or prognosis of the patient or his/her family, which comprises the following steps:

(1) detecting multiple microsatellite loci as described in embodiment 15 simultaneously;

(2) determining the stability status of microsatellite loci in the sample according to the method of any one of embodiments 15-18;

(3) obtaining the detection results of the one or more of disease-related genes according to the sequencing results;

(4) providing clinical guidance on the risk control, treatment and/or prognosis of the patient or his/her family by combining the results of the above steps (2) and (3).

Preferably, in the method for detecting microsatellite stability status and disease-related gene variations in patients based on next-generation high-throughput sequencing to provide clinical guidance on the risk control, treatment and/or prognosis of the patient or family provided by the present disclosure, the disease is cancer, preferably colorectal cancer (such as bowel cancer), gastric cancer or endometrial cancer.

In yet another aspect, the present disclosure further relates to a kit used for one of various methods of the present disclosure, which comprises a reagent for detecting the multiple microsatellite loci.

In another aspect, the present disclosure further provides a device for determining microsatellite marker loci used in the detection of microsatellite stability status in a plasma sample, characterized in that the device comprises:

a module for reading sequencing data for use in reading the sample sequencing data obtained and stored in the sequencing equipment,

a module for detecting microsatellite marker loci for use in analysis and detection of all microsatellite loci in the sequencing region in the sample from the sample sequencing data,

a module for determining the length type of repetitive sequences for use in counting the number of reads of each length types of different repetitive sequence through the sample sequencing data read using the module for reading sequencing data for any one of the microsatellite loci $i$,

a module for determination, which is used in determining whether any one of the microsatellite loci $i$ is a microsatellite marker locus, the module for determination comprising a first analysis module, a second analysis module, and a third analysis module,

the first analysis module is used to determine the length characteristics of the locus repetitive sequence under microsatellite stable (MSS), and determine whether the number of corresponding reads in the MSS sample is greater than 75% of the total number of reads supported by the locus, wherein length characteristics of MSS is a minimum range of continuous length, and it is recorded as "+" if a positive result is obtained and it is recorded as "-" if a negative result is obtained,

the second analysis module is used to determine the length characteristics of the locus repetitive sequence under microsatellite instability-high (MSI-H), wherein the length characteristics of MSI-H is a range of continuous length that is highly differentiated in MSS and MSI-H samples, and determine that a) whether the total number of reads supported within the range of continuous length is less than 0.2% of the total number of reads at the locus in the MSS sample, which is recorded as "+" if a positive result is obtained and recorded as "-" if a negative result is obtained, and b) whether the reads account for more than 50% of the total number of reads at the locus in the MSI-H sample, which is recorded as "+" if a positive result is obtained and recorded as "-" if a negative result is obtained,

the third analysis module is used to analyze the results of the first analysis module and the second analysis module, and determine the microsatellite locus $i$ as a microsatellite marker locus if three positive results are obtained, i.e. three "+"s.

Preferably, in the device for determining microsatellite marker loci used in the detection of microsatellite stability status in a plasma sample provided by the present disclosure, the sample includes a sample from normal white blood cells and tissues from cancer patients, and the cancer is preferably colorectal cancer (such as bowel cancer), gastric cancer or endometrial cancer. More preferably, the microsatellite locus determined by the device as described above comprises one or more of the 8 microsatellite loci described in Table 1.

In one embodiment, in the device for determining microsatellite marker loci used in the detection of microsatellite stability status in a plasma sample provided by the present disclosure, the detection of microsatellite stability status is used for non-invasive diagnosis, prognostic evaluation, selection of treatment or genetic screening of cancer, preferably colorectal cancer (such as bowel cancer), gastric cancer or endometrial cancer.

In yet another aspect, the present aspect further relates to a device for determining the stability status of microsatellite loci through a plasma sample of a cancer patient based on the next-generation high-throughput sequencing method, characterized in that the device comprises:

a module for reading sequencing data for use in reading the sample sequencing data obtained and stored in the sequencing equipment,

a module for determining the length characteristics of repetitive sequences for use in analyzing the length characteristics of repetitive sequences of multiple microsatellite loci in a plasma sample and an MSS plasma sample as the reference sample from the sample sequencing data, the multiple microsatellite loci comprising one or more of microsatellite loci selected from the 8 microsatellite loci shown in Table 1;

a module for calculating enrichment index for use in calculating enrichment index Zscore for the microsatellite loci;

a module for calculating the microsatellite status index for use in summing the enrichment index Zscore of all microsatellite loci to result in the index MSscore for judging the status of microsatellites of the sample;

a module for calculating the threshold for use in calculating the mean and standard deviation SD of the MSscore of the MSS plasma sample as the reference sample, with mean + 3SD as the threshold cutoff;

a template for determining the stability status of micro satellite loci for use in comparing index MSscore with threshold cutoff, and determining the sample as MSI-H when MSscore > cutoff and determining the sample as MSS when MSscore ≤ cutoff for a plasma sample from a cancer patient.

In one embodiment, in the device of determining the stability status of microsatellite loci through the plasma samples of cancer patients based on the next-generation high-throughput sequencing method, characterized in that the Zscore is evaluated by $H_s$,

which is evaluated by $H_s = - \log (P_s(X > k_s))$,

and

$$P(X = k) = \frac{\binom{K}{k}\binom{N - k}{n - k}}{\binom{N}{n}}$$

wherein N is the total number of reads in the repetitive sequence length set for MSI-H status and MSS status, K is the total number of reads in the repetitive sequence length set for MSI-H status, and N - K is the total number of reads in the repetitive sequence length set for MSS status, and correspondingly, $n$ and $k$ are the number of respective reads in the sample to be tested, respectively.

Preferably, in the device for determining stability status of microsatellite loci as described above, MSscore is calculated based on the following formula:

$$MSscore = \sum_{s \in \text{markers}} \frac{H_s - \underset{\text{MSS\_Sample } s}{mean}(H_s)}{\underset{\text{MSS\_Sample } s}{sd}(H_s)}$$

More preferably, in the device for determining stability status of microsatellite loci as described above, the disease is cancer, preferably colorectal cancer (such as bowel cancer), gastric cancer, or endometrial cancer.

**Brief Description of the Drawings**

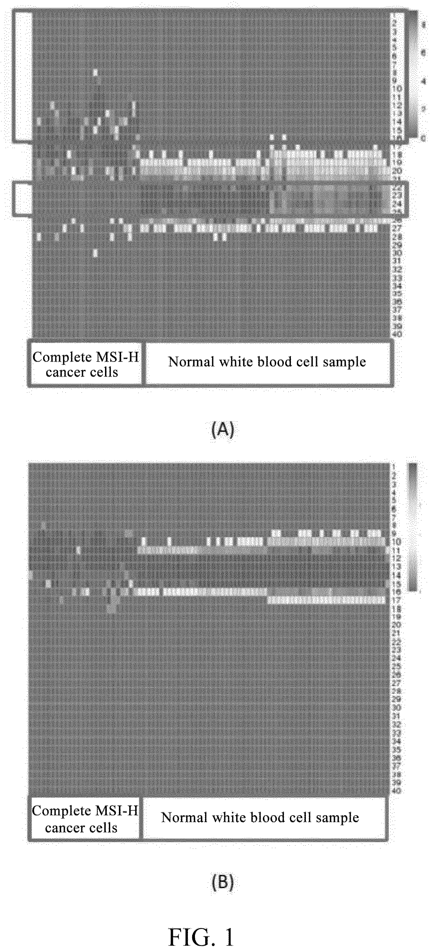

Figure 1. (A) The distribution of the numbers of reads of each repetitive sequence length of the microsatellite marker locus bMS-BR1 in complete MSI-H cancer cells and white blood cell samples. The blue box indicates that the characteristic range of MSS at this locus is 22-25 bp, and the red box indicates that the characteristic range of MSI-H at this locus is <16 bp. (B) The distribution of the numbers of fragments of each repetitive sequence length in complete MSI-H cancer cells and white cell samples of non-marker loci. Although the length of the repetitive sequence at this locus has been shortened by about 2 bp, this difference is not sufficient to distinguish from the fluctuation of the capture of white blood cells under the condition that the ctDNA content of the tumor is very small. There is not such a type of repetitive sequence length that only occurs frequently in MSI-H samples.

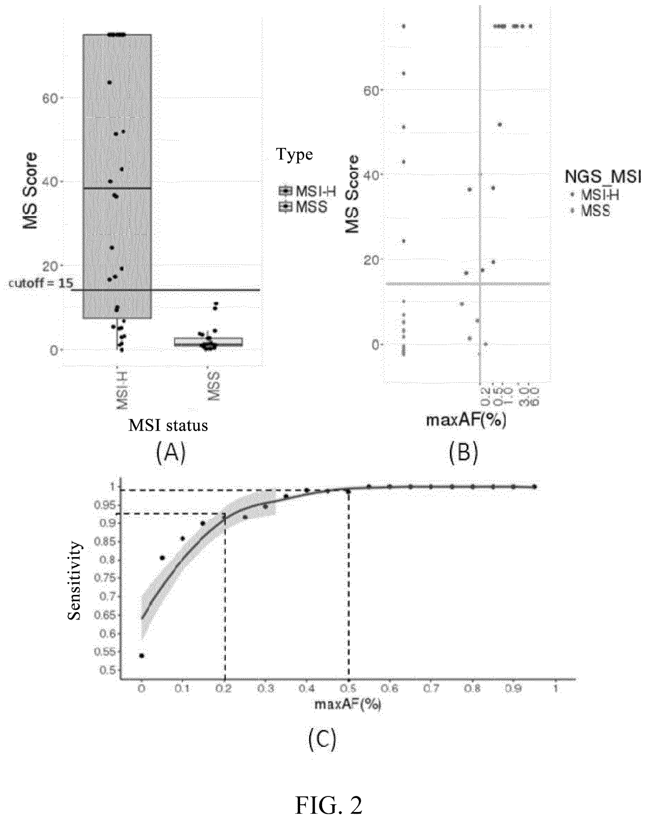

Figure 2. Effect of bMSISEA detection. (A) Distribution of MSscore of 127 cases of colorectal cancer plasma samples. The MS status is determined by the matched tissues. A total of 44 cases of MSI-H samples and 83 cases of MSS samples are included. When the MSscore is higher than cutoff=15, the plasma sample is determined as MSI-H, and when the MSscore is less than or equal to 15, it is determined as MSS; (B) Correlation of 44 cases of MSI-H sample maxAF and MSscore; red dots indicate MSscore>15, and the sample is determined as MSI-H, and blue dots indicate MSscore does not suffice the threshold, and the sample is determined as MSS; (C) Correlation between detection sensitivity and maxAF based on simulated samples. The results are based on 350 simulated samples with different ctDNA content gradients. The horizontal axis indicates that only samples with maxAF greater than the corresponding value are counted. The vertical axis is the detection sensitivity of MSI-H. When maxAF>0.2%, the sensitivity of MSI-H detection is higher than 93%, and when maxAF>0.5%, the sensitivity is higher than 98%.

**Detailed Description of the Invention**

This disclosure provides a method for detecting the microsatellites stability and disease-related genes through plasma for the first time based on next-generation sequencing, and based on such detection method, MSI loci for detecting cancer, preferably colorectal cancer (such as bowel cancer), gastric cancer or endometrial cancer with high sensitivity and specificity are obtained.

In addition, the present disclosure establishes a method for determination of microsatellite marker loci capable of detecting microsatellite status based on plasma samples. The present disclosure also realizes the simultaneous detection of multiple microsatellite loci and multiple disease-related genes in the sample, which can give more comprehensive conclusions and suggestions on prognosis, treatment, investigation, etc. of the detected sample.

This disclosure thus provides a method for detection of MSI in plasma for the first time, and compared with MSI detection in tissues, the plasma MSI detection of this disclosure is non-invasive, real-time and non-tissue specific. At the same time, the method of the present disclosure can complete the detection of microsatellite status in plasma samples with very low ctDNA content, filling the gap in the detection of microsatellite status through plasma samples, and can achieve high accuracy for samples with ctDNA content higher than 0.4%. It has fast detection speed, does not rely on matching white blood cell samples, has lower prices, has faster detection and can determine the microsatellite stable (MS) status of the sample with high sensitivity and high specificity.

In addition, the detection method of the present disclosure can also be used for non-invasive diagnosis, prognostic evaluation, or selection of treatment for patients with cancer, preferably colorectal cancer (such as bowel cancer), gastric cancer or endometrial cancer.

In addition, this disclosure also provides a device for determining microsatellite marker loci used in the stability status detection of microsatellites in plasma samples and a device for determining stability status of microsatellite loci from plasma samples of cancer patients based on the next-generation high-throughput sequencing method.

The inventors found that for samples of microsatellite instability-high, their microsatellite loci cause the expansion or contraction of a large number of repetitive sequences due to incorrect DNA duplication. In this regard, by comparing the length types of the repetitive sequence of the reads of the MSI-H tissue samples and the normal white blood cell samples, we can find the length type of repetitive sequence that appears in a large number in the MSI-H tissue sample but rarely appears in the normal white blood cell sample as the characteristic of the length of the repetitive sequence at the locus under the MSI-H status.

The specific criteria for selection of marker loci are as follows: a) The number of reads within the length range of repetitive sequences in the MSS sample is less than 0.2% of the total number of reads at the locus; and b) the number of reads in the range in the MSI-H sample occupies more than 50% of the total number of reads at the locus. At the same time, the length range is defined as the characteristics of the length of repetitive sequences at the locus under the MSI-H status. Through the above two conditions, the method ensures that even with extremely low ctDNA content, the reads covering the length characteristics of MSI-H are almost entirely derived from tumor DNA.

Based on this choice, the inventors screened out 8 microsatellite marker loci (see Table 1 for details).

## Table 1 Information for microsatellite detection marker loci

| Locus Identity | Chromosome | Left-mer | Homopolymer | Right-mer | MSI-H Mode | MSS Mode | Mean of HS score | Standard derivation of HS score |
|---|---|---|---|---|---|---|---|---|
| bMS-BR1 | 11: 102193508-102193534 | CTGGT | 26[A] | GCCAC | 1-16 | 22-25 | 0.63 | 1.29 |
| bMS-BR2 | 15: 91303186-91303202 | AAGAC | 16[T] | AGTGA | 1-11 | 15-16 | 0.97 | 0.85 |
| bMS-BR3 | 2: 47641559-47641586 | CAGGT | 27[A] | GGGTT | 1-15 | 21-25 | 0.52 | 1.15 |
| bMS-BR4 | 14: 23652346-23652367 | TTGCT | 21[A] | GGCCA | 1-15 | 21-24 | 2.90 | 13.99 |
| bMS-BR5 | 11: 108114661-108114676 | AATAA | 15[T] | AAGAA | 1-12 | 15 | 0.16 | 0.33 |
| bMS-BR6 | 2: 39573062-39573089 | GTCTC | 27[A] | GAGTG | 1-17 | 22-27 | 1.84 | 4.19 |
| bMS-BR7 | 11: 125490765-125490786 | GAAGA | 21[T] | AATAT | 1-15 | 19-21 | 1.77 | 4.75 |
| bMS-BR8 | 7: 116381121-116381137 | TGGTG | 16[T] | GGTTT | 1-11 | 15-16 | 0.72 | 4.51 |

This disclosure is based on the next-generation high-throughput sequencing method to determine the stability status of microsatellite loci in plasma samples from cancer patients, that is, the main strategy of the applicant's microsatellite instability plasma detection technology named bMSISEA is to first search for marker loci with completely different coverage of reads under MSI-H and MSS statuses and describe the main length types of reads supported by the loci under both statuses. Through the enrichment analysis of the characteristics of reads at each marker locus with respect to MSI-H status, the instability status is evaluated, and then the microsatellite status of the sample is determined.

The method for determining the stability status of microsatellite loci in plasma samples from cancer patients in this disclosure comprises the following steps: 1) data preparation, including sample preparation, detection of the microsatellite locus in the sequencing region, and statistics on the length types of repetitive sequences at the locus; 2) screening of the marker locus and description of locus characteristics; 3) enrichment analysis of the microsatellite instability characteristics; 4) evaluation of the average fluctuation level of the enrichment index at each locus; 5) construction of the MS score based on the relative level of the enrichment index of the plasma sample to be tested, and then determination of the MS status of the sample.

At the same time, this disclosure provides the following examples to help understand the present disclosure, and the true scope of the present disclosure is given in the appended claims. It should be understood that the presented method can be modified without departing from the spirit of the disclosure.

**Examples**

**1. Data preparation: gene panel detection is carried out based on Next-generation sequencing method with the specific steps as follows.**

The capture steps of tissue samples are as follows: Tumor tissue and paracancerous normal tissue DNA were extracted using QIAamp DNA FFPE tissue kit (QIAGEN: 56404). Accurate quantification was performed using dsDNA HS assay kits (ThermoFisher: Q32854) with the Qubit 3.0 fluorometer. The extracted DNA was physically fragmented into 180-250 bp fragments using a sonicator Covaris M220 (Covaris: PN500295), and then repaired, phosphorylated, added deoxyadenine at the 3' end, and ligated with a linker. The DNA ligated to the amplification linker was then purified using Agencourt AMPure XP paramagnetic beads and pre-amplified using PCR polymerase, and the amplified product was hybridized with Agilent's custom multiplexed biotin-labeled probe set (the gene panel design includes sequences of exons and partial intron regions of 41 genes). After the successfully hybridized fragments were specifically eluted, and amplified by PCR polymerase, quantification and fragment length distribution determination were performed, and Next-generation sequencing was performed using an IlluminaNovaseq 6000 sequencer (Catalog No. 20012850) with a sequencing depth of 1000X.

The capture steps of blood samples are as follows: firstly, the nucleic acid extraction reagent was employed to extract the free DNA in the plasma and the genomic DNA in the matched peripheral blood leukocyte, and the leukocyte genomic DNA is fragmented. Then, the whole genome pre-library was prepared by steps of addition of linkers, PCR amplification and the like, which was hybridized with the RNA probe of a specific sequence labeled with biotin to specifically capture part of the exon and intron regions (full coding region, exon-intron junction region, UTR region and promoter region) of 41 genes in the human genome. The DNA fragments captured by the probes were enriched with streptavidin magnetic beads, and the enriched DNA fragments were used as templates for amplification, resulting in the final library. After quantification and quality control of the final library, the final library was subject to high-throughput sequencing with an IlluminaNovaSeq gene sequencer, with a sequencing depth of 15000X.

Finally, the measured sequences were aligned with the human genome sequence (version hg19) using BWA version 0.7.10, GATK 3.2 was used for local alignment optimization, VarScan 2.4.3 was used for mutation calling, and ANNOVAR and SnpEff 4.3 were used for mutation annotation. For mutation calling, loci with low coverage will be removed by VarScanfpfilter (tissue: below 50x, plasma: below 500x, and white blood cell: below 20x); for indels and single point mutations, at least 5 and 8 mutated reads are required respectively.

**2. Statistics of length types of the repetitive sequences at microsatellite loci based on next-generation sequencing (NGS) data**

Only the binary sequence alignment (BAM) file of the cancer plasma sample is required during the microsatellite instability detection algorithm bMSISEA detection. BAM files of following samples are also required during the baseline construction process: sufficient matched MSI-H cancer tissue and normal samples (number greater than 50), sufficient white blood cell samples (number greater than 100), and sufficient MSS plasma samples (number greater than 100).

MSIsensor (v 0.5) software was firstly employed in this method to obtain all the microsatellite loci with a length greater than 10 and repetitive sequences of 1 in the sequencing coverage region, and the number of reads covered by the repetitive sequence of each length type at the microsatellite loci was calculated.

The method for counting the number of reads covered by each length type of the locus by MSIsensor is as follows: For each microsatellite locus, its position information and sequences at both ends were first searched for in the human genome, and all sequences with the intermediate repetitive sequence length of 1 to L-10 bp connected by the sequences at both ends were constructed as a search dictionary with L as the length of reads. For example, a single base microsatellite locus on chromosome 1 (14T, T is a repeating base, 14 is the number of repetitions), the sequences at both ends are ATTCC and GCTTT, and the constructed search dictionary comprises ATTCCTGCTTT (repeat length is 1), ATTCCTTGCTTT (repeat length is 2), ATTCCTTTGCTTT (repeat length is 3), and so on. Paired reads with at least one end located within 2 kb of the locus were extracted from the BAM file of the sample and aligned to sequences in the search dictionary of the locus. The number of the reads covering different lengths in the search dictionary was counted and a histogram of the number of the reads covering all length types of the locus was constructed.

### 3. Screening of marker loci for microsatellite instability

3.1 Length characteristics of the repetitive sequence at the locus under MSS status

For the microsatellite loci of normal samples, a high probability of coverage of the reads is on one or two length types of repetitive sequences corresponding to the sample genotype. The length type of repetitive sequences that is likely to appear in the reads at each locus under normal status is described based on the white blood cell sample in this step as the characteristics of the repetitive sequence length at the locus under the MSS status. For each white blood cell sample at each locus, the minimum range of continuous lengths is searched for so that the number of corresponding reads is greater than 75% of the total number of reads supported by the locus. This continuous length range is referred as the peak region of the sample at the locus. For each locus, the length range of the repetitive sequences selected as the peak region in at least 25% of the white blood cell samples is used as the characteristics of the length of the repetitive sequences at the locus under the MSS status.

3.2 Characteristics of the length of the repetitive sequences at the locus under the MSI-H status and selection of marker locus

For samples of microsatellite instability-high, their microsatellite loci cause the expansion or contraction of a large number of repetitive sequences due to incorrect DNA duplication. Here, we focus on the phenomenon of sequence contraction of long repetitive sequences. The type length of repetitive sequences under MSI-H status that is different from that under the normal status occurring in the large number of reads is described in this step based on matched MSI-H cancer tissue and adjacent normal tissue samples as the characteristics of the repetitive sequence length at the locus under the MSI-H status. Since the cancer tissue sample is a mixture of cancer cells and normal cells, the first step of the method is to estimate the proportion of tumor cells in the sample. The specific method is as follows: the number of reads of the length type of repetitive sequences at the locus corresponding to the MSS status at each locus was counted in the cancer tissue and the adjacent normal tissue, and a linear model was established assuming that the reads for the MSS status in the cancer tissue sample are completely derived from the normal cells therein, to estimate the proportion of tumor cells: $u$. In the second step, the total numbers of reads of the cancer tissue and the matched normal tissue were normalized, and then $u$ times of the corresponding data of the matched normal tissue were correspondingly subtracted from the number of reads for each the length of the repetitive sequences at each locus in the cancer tissue, thereby estimating the complete repetitive sequence length statistics of MSI-H cancer cells.
For all microsatellite loci, loci with the following characteristics are selected as the marker loci of bMSISEA based on the statistical data of the repetitive sequence length of complete MSI-H cancer cells, and the length range of repetitive sequences is used as the characteristics of the repetitive sequence at the locus under the MSI-H status: the number of reads supported by the length range of repetitive sequences in the MSS sample is less than 0.2% of the total number of reads at the locus, and accounts for more than 50% of the total number of reads at the locus in the MSI-H sample. The above two conditions ensure that even with extremely low ctDNA content, the reads covering the length characteristics of MSI-H are almost entirely derived from cancer DNA.
8 microsatellite detection marker loci screened out according to the above method for microsatellite status detection are listed in Table 1. The marker locus bMS-BR1 is shown in Figure 1 (A). Therein, the characteristic length of the repetitive sequences at the locus under the MSS status is in the range of 22-25 bp, and the characteristic length of the MSI-H is in the range of 1-16 bp. The coverage feature maps of a non-marker locus in two types of samples are shown in Figure 1(B). Although the length of the repetitive sequence at this locus under MSI-H status has been shortened by about 2 bp compared with MSS sample, this variation cannot be distinguished from the fluctuation of the capture of white blood cells under the condition that the ctDNA content of the tumor is very small, which does not meet the screening conditions of the marker loci and cannot be used to determine the microsatellite status of the sample.

### 4. Enrichment analysis of MSI characteristics

For each marker locus, the plasma samples were subjected to enrichment analysis for MSI-H characteristics with the number of reads corresponding to the length characteristics set of the normal white blood cell samples under the MSS and MSI-H statuses as the background. The total numbers of reads corresponding to the length set of the repetitive sequences under the MSS status and MSI-H status were calculated based on a large number of normal white blood cell samples and were denoted as $K$ and $N\text{-}K$, respectively. For plasma samples, the numbers of reads, $k$ and $n\text{-}k$, corresponding to the length set of the repetitive sequences under the MSS status and MSI-H status were also calculated. If the sample status is MSS, the characteristics of read are consistent with the white blood cell sample status and conform to the hypergeometric distribution

$$P(X = k) = \frac{\binom{K}{k}\binom{N-k}{n-k}}{\binom{N}{n}}$$

Therefore, the enrichment index of the locus can be evaluated by $H_s$, $H_s = -\log(P_s(X > k_s))$.

Furthermore, based on a large number of MSS plasma samples, the fluctuation range of the enrichment index of each locus is obtained. For a plasma sample to be tested, the Zscore of the enrichment index of each locus is calculated based on the fluctuation level, and all Zscores are summed to obtain the index MSscore for determining the microsatellite status of the sample.

$$MSscore = \sum_{s \in \text{markers}} \frac{H_s - \underset{\text{MSS\_Sample s}}{mean}(H_s)}{\underset{\text{MSS\_Sample s}}{sd}(H_s)}$$

Taking the bMS-BR1 locus as an example, the total number $K$ of reads with repetitive sequence length ranging from 1-16 bp is 504 based on 100 WBC samples, and the total number $N$ of reads with length ranging from 1-16 bp or 22-25 bp is 190588. For a sample to be tested, the total number $k$ of reads of the repetitive sequence at the locus in the length range of 1-16 bp is 65, and the total number $n$ of reads of 1-16 bp or 22-25 bp is 1308, such that $H_s = -\log(P_s(X > k_s)) = -\log(P_s(X > 65)) = 140.6$. Furthermore, the fluctuation level of $H_s$ is evaluated based on the MSS plasma sample, as shown in Table 1, $mean(H_s) = 0.63$, sd $(H_s) = 1.29$, resulting in the Zscore value of this MSS_Sample s MSS_Sample s locus of 108.6. The calculation method for other loci is as described above. Finally, all Zscores are summed up to result in the final MSscore of this locus of 355.3. The suspected pathogenic system frameshift mutation p.D214fs of MLH1, and pathogenic/suspected pathogenic mutations including PIK3CA, KRAS, PTEN, and mutations with unknown pathogenic information including BRCA2, STK11, PMS1, and benign mutations of other genes involved in the kit were detected in the sample at the same time.

### 5. Determination of the microsatellite status of cancer samples

For a plasma sample, based on the MSSCore value of the MSS plasma sample, its average mean and standard deviation SD are calculated, and mean + 3SD is used as the threshold cutoff. When Msscore > cutoff, the sample is determined as MSI-H, and when MSscore ≤ cutoff, the sample is determined as MSS.

### 6. Results for detection of plasma for bMSISEA microsatellite instability

We performed NGS detection including mutation and microsatellite detection on 127 real clinical colorectal cancer plasma samples based on the 8 microsatellite marker loci listed in Table 1 and detection kits using bMSISEA microsatellite detection technology. The microsatellite status of the sample is double confirmed by IHC and NGS-MSI technology to comprise 44 MSI-H samples and 83 MSS samples based on the matched tissue samples of the corresponding patient. The method of tissue detection is as follows: the microsatellite status of the sample is determined through 22 marker loci by the NGS detection method based on the difference in the length of the repetitive sequences. For each marker locus, the method evaluates the length range of repetitive sequences of reads that appear collectively under the MSS status, and evaluates the percentage change of the reads in this range to the total number of reads at the locus. With mean - 3*sd* as the threshold, if the ratio at the locus as described above is less than the threshold value, the locus is determined to be an unstable locus. If the total number of unstable loci is less than 15% of the number of total loci, the sample is determined as MSS, and if it is higher than 40%, the sample is determined as MSI-H, and if it is between the two, it is determined as MSI-L. The detection method can be referred to Chinese Patent Application No. 201710061152.6. In addition, IHC assessment was also completed through the histopathological section. MMR proteins, including the expression profile of MLH1, PMS2, MSH2, and MSH6 proteins were detected by the IHC method using immuno-histochemical methods. If one of the proteins is missing, it is determined as dMMR, and if there is no protein missing, it is determined as pMMR. Patients with dMMR usually have MSI-H due to abnormal mismatch repair mechanisms.

By comparing the detection results of these 127 plasma samples based on the bMSISEA results with those of matched tissues thereto, the sensitivity and specificity of the bMSISEA method are shown in Table 2.

Table 2. bMSISEA detection results based on 127 cases of colorectal cancer plasma (based on tissue detection results)

| | | Microsatellite status based on tissue detection | | Detection Indicator |
|---|---|---|---|---|
| | | MSI-H | MSS | |
| Microsatellite status based on plasma detection | MSI-H | 23 | 0 | PPV 100% |
| | MSS | 21 | 83 | NPV 79.8% |
| Detection Indicator | | Sensitivity 52.3% | Specificity 100% | Accuracy 83.5% |

When ctDNA (maxAF>0.2%), the accuracy of plasma MSI detection reaches 98.5%.

| | | Microsatellite status based on tissue detection | | Detection Indicator |
|---|---|---|---|---|
| | | MSI-H | MSS | |
| Microsatellite status based on plasma detection | MSI-H | 15 | 0 | PPV 100% |
| | MSS | 1 | 52 | NPV 98.1% |
| Detection Indicator | | Sensitivity 93.8% | Specificity 100% | Accuracy 98.5% |

*The microsatellite status results based on tissue detection are double confirmed by NGS and IHC methods. Among the detection indicators, sensitivity: sensitivity; specificity: specificity; PPV: positive predictive value; NPV: negative predictive value; accuracy: accuracy. The calculation method is as follows:

$$\mathbf{sensitivity} = \frac{TP}{TP + FN}$$

$$\mathbf{specificity} = \frac{TN}{TN + FP}$$

$$\mathbf{PPV} = \frac{TP}{TP + FP}$$

$$\mathbf{NPV} = \frac{TN}{TN + FN}$$

$$accuracy = \frac{TP + FN}{TP + TN + FP + FN}$$

wherein TP, TN, FP, FN represent the number of samples which are true positive (the detection results of tissue and plasma are both MSI-H), true negative (the detection results of tissue and plasma are both MSS), false positive (the detection result of tissue is MSS, and the detection result of plasma is MSI-H), false negative (the detection result of tissue is MSI-H, and the detection result of plasma is MSS), respectively.

It can be seen from Table 2 that the specificity of MSI-H detection based on plasma samples is 100%. When all samples are included without screening, the overall sensitivity of detection is only 52.3% and the accuracy is 83.5% because most samples have extremely low ctDNA content. In contrast, when only plasma samples that meet max-AF>0.2% (ctDNA>0.4%) are screened, the sensitivity of detection is 93.8%, and the accuracy is 98.5%. In fact, when only samples with maxAF>0.5% in this group of samples are selected, the detection accuracy is 100%. It can be seen that on the basis of ensuring the specificity of detection, bMSISEA has a sufficiently high detection sensitivity when the plasma contains sufficient content of ctDNA.

In addition, a more detailed detection result is shown in Figure 2. Figure 2(A) shows the MSscore distribution based on MSI detection of 127 colorectal cancer plasma samples. Based on the bMSISEA method, 83 MSS samples had MSscore less than 15, with a specificity of 100%. 23/44 MSI-H samples had MSscore greater than 15, with the sensitivity of 52.3%. Taking into account the difference in ctDNA content between samples, Figure 2(B) describes the correlation between maxAF and MSscore of MSI-H samples. Only considering samples with maxAF>0.2%, 15/16 cases of MSI-H samples had MSscore greater than 15, with accuracy of 93.8%.

**7. Influence of ctDNA content in plasma on detection sensitivity confirmed by simulation experiments**

Since the content of ctDNA in plasma is generally extremely low, the detection sensitivity will be affected by the content of ctDNA. Therefore, based on real clinical plasma and white blood cell samples, a set of 350 simulated samples with different ctDNA content gradients were constructed in this experiment to evaluate the sensitivity of detection of microsatellite instability based on plasma sample by the method under different ctDNA content. Here, the ctDNA content of the cancer sample can be evaluated by the maximum somatic gene mutation frequency (maxAF) of the sample.

We selected 18 pairs of matched plasma and white blood cell samples, mixed bam files of plasma and white blood cell samples in proportion based on the maxAF of the plasma samples and re-sampled to the original plasma sample, simulating 350 samples with different ctDNA content gradients to evaluate the sensitivity level of plasma samples containing different ctDNA contents. The simulated samples employed the same mutation detection process as the real clinical samples for mutation detection to determine the maxAF level. As shown in Figure 2(C), the horizontal axis is to count only the samples whose maxAF is greater than the threshold, and the vertical axis is the detection sensitivity of MSI-H. When maxAF>0.2%, the detection sensitivity of MSI-H is higher than 93%, and when maxAF>0.5%, the sensitivity is higher than 98%. Although the detection of MSI-H is limited when the content of ctDNA is too low, when the content of ctDNA reaches the stable detection range (maxAF>0.2%), the bMSISEA method can determine the microsatellite stable (MS) status of the sample with high accuracy and sensitivity, which provides the possibility of non-invasive detection of MS status in plasma.

Therefore, for plasma samples with maxAF>0.2% (approximately corresponding to ctDNA content higher than 0.4%), sensitivity that matches the tissue detection and extremely high specificity can be obtained by the bMSISEA method. Compared with MSI detection in tissues, the plasma MSI detection of this disclosure has the unique advantages of liquid biopsy, including non-invasive diagnosis, non-tissue specificity, and detection of multiple lesions. The bMSISEA method does not rely on matched white blood cell samples to detect mutations while determining the microsatellite status of the sample at a lower price and faster speed.

**Claims**

1.  A biomarker panel comprising one or more of 8 microsatellite loci as shown in Table 1.

2.  A biomarker panel comprising a combination of microsatellite loci and one or more of genes, wherein the microsatellite loci comprise the 8 microsatellite loci shown in claim 1 or a combination of any one or more, wherein the one or more of genes are any one or more of the following 41 genes: AKT1, APC, ATM, BLM, BMPR1A, BRAF, BRCA1, BRCA2, CDH1, CHEK2, CYP2D6, DPYD, EGFR, EPCAM, ERBB2, GALNT12, GREM1, HRAS, KIT, KRAS, MET, MLH1, MSH2, MSH6, MUTYH, NRAS, PDGFRA, PIK3CA, PMS1, PMS2, POLD1, POLE, PTCH1, PTEN, SDHB, SDHC, SDHD, SMAD4, STK11, TP53, UGT1A1.

3. A kit for the detection of microsatellite stability in a plasma sample, **characterized in that** the kit comprises a detection reagent for the biomarker panel according to claim 1 or 2.

4. A kit for use in the non-invasive diagnosis, prognostic evaluation, selection of treatment or genetic screening of cancer, preferably colorectal cancer (such as bowel cancer), gastric cancer or endometrial cancer, **characterized in that** the kit comprises a detection reagent for the biomarker panel according to claim 1 or 2.

5. The kit of claim 3 or 4, wherein the plasma sample is a cancer plasma sample, preferably a colorectal cancer plasma sample, such as a bowel cancer plasma sample, a gastric cancer plasma sample, and an endometrial cancer plasma sample.

6. The kit of claim 3, wherein the microsatellite stability comprises types of microsatellite instability-high (MSI-H), microsatellite instability-low (MSI-L), and microsatellite stable (MSS).

7. The kit of any one of claims 3-6, wherein the detection reagent is a reagent for performing next-generation high-throughput sequencing (NGS).

8. Use of the biomarker panel of claim 1 or 2 in detection of the microsatellite stability in a plasma sample.

9. The use of claim 8, wherein the plasma sample is a cancer plasma sample, preferably a colorectal cancer plasma sample, such as a bowel cancer plasma sample, a gastric cancer plasma sample, and an endometrial cancer plasma sample.

10. The use of claim 9, wherein the microsatellite stability comprises types of microsatellite instability-high (MSI-H), microsatellite instability-low (MSI-L), and microsatellite stable (MSS).

11. Use of the biomarker panel of claim 1 or 2 in the non-invasive diagnosis, prognostic evaluation, selection of treatment or genetic screening of cancer, preferably colorectal cancer (such as bowel cancer), gastric cancer or endometrial cancer.

12. A method for determining microsatellite marker loci that can be used in the detection of microsatellite instability in a plasma sample, which comprises the following steps:

> 1) detecting the microsatellite loci in the sequencing region of the sample;
> 2) counting the number of reads of each length types of different repetitive sequence counted by NGS data for any one of the microsatellite loci *i;*
> 3) determining the length characteristics of the locus repetitive sequence under microsatellite stable (MSS) and the length characteristics of the locus repetitive sequence under microsatellite instability-high (MSI-H) for any one of the microsatellite loci; wherein the length characteristics of MSS is a minimum range of continuous length, such that the number of corresponding sequencing fragments in the MSS sample is greater than 75% of the total number of reads supported by the locus; the length characteristics of MSI-H is a range of continuous length that is highly differentiated in MSS and MSI-H samples, such that a) the total number of reads supported by this range is less than 0.2% of the total number of reads at the locus in the MSS sample, and b) accounts for more than 50% of the total number of reads at the locus in the MSI-H sample,

> the microsatellite locus with the above characteristics being the detection marker of microsatellite locus.

13. The method of claim 12, wherein the sample includes a sample from normal white blood cells and tissues from cancer patients, and the cancer is preferably colorectal cancer (such as bowel cancer), gastric cancer or endometrial cancer.

14. The microsatellite locus determined by the method of claim 12, which comprises one or more of the 8 microsatellite loci described in Table 1.

15. The method of any one of claims 12-14, wherein the detection of microsatellite instability is used for non-invasive diagnosis, prognostic evaluation, selection of treatment or genetic screening of cancer, preferably colorectal cancer (such as bowel cancer), gastric cancer or endometrial cancer.

**16.** A method for determining the stability status of microsatellite loci through a plasma sample of a cancer patient based on the next-generation high-throughput sequencing method, which comprises the following steps:

1) determining the length characteristics of repetitive sequences of multiple microsatellite loci in a plasma sample and an MSS plasma sample as the reference sample based on the next-generation sequencing method, the multiple microsatellite loci comprising one or more of microsatellite loci selected from the 8 microsatellite loci shown in Table 1;

2) calculating its corresponding enrichment index Zscore for any one of microsatellite loci described in 1);

3) summing the enrichment index Zscore of all microsatellite loci to result in the index MSscore for judging the status of microsatellites of the sample;

4) calculating the mean and standard deviation SD of the MSscore of the MSS plasma sample as the reference sample, with mean + 3SD as the threshold cutoff;

5) determining the sample as MSI-H when MSscore > cutoff and determing the sample as MSS when MSscore ≤ cutoff for a plasma sample from a cancer patient.

**17.** The method of claim 16, wherein the Zscore is evaluated by $H_s$, evaluated by $H_s = - \log(P_s(X > k_s))$, and

$$P(X = k) = \frac{\binom{K}{k}\binom{N-k}{n-k}}{\binom{N}{n}}$$

wherein N is the total number of reads in the repetitive sequence length set for MSI-H status and MSS status, K is the total number of reads in the repetitive sequence length set for MSI-H status, and N - K is the total number of reads in the repetitive sequence length set for MSS status, and correspondingly, $n$ and $k$ are the number of respective reads in the sample to be tested, respectively.

**18.** The method of claim 16, wherein MSscore is calculated based on the following formula:

$$MSscore = \sum_{s \in \text{markers}} \frac{H_s - \underset{\text{MSS\_Sample } s}{mean}(H_s)}{\underset{\text{MSS\_Sample } s}{sd}(H_s)}$$

**19.** The method of claim 16, wherein the cancer is colorectal cancer (such as bowel cancer), gastric cancer, or endometrial cancer.

**20.** A method for detecting microsatellite instability and disease-related gene variations in patients based on next-generation high-throughput sequencing to provide clinical guidance on the risk control, treatment and/or prognosis of the patient or family, which comprises the following steps:

(1) detecting multiple microsatellite loci as described in claim 16 simultaneously;

(2) determining the stability status of microsatellite loci in the sample according to the method of any one of claims 5-8;

(3) obtaining the detection results of the one or more of disease-related genes according to the sequencing results;

(4) providing clinical guidance on the risk control, treatment and/or prognosis of the patient or family by combining the results of the above steps (2) and (3).

**21.** The method of claim 20, wherein the disease is cancer, preferably colorectal cancer (such as bowel cancer), gastric cancer or endometrial cancer.

**22.** A kit used for the method of any one of claims 12-20, which comprises a reagent for detecting the multiple microsatellite loci.

23. A device for determining microsatellite marker loci used in the detection of microsatellite instability in a plasma sample, **characterized in that** the device comprises:

the module for reading sequencing data for use in reading the sample sequencing data obtained and stored in the sequencing equipment,

the module for detecting microsatellite marker loci for use in analysis and detection of all microsatellite loci in the sequencing region in the sample from the sample sequencing data,

the module for determining the length type of repetitive sequences for use in counting the number of reads of each length types of different repetitive sequence through the sample sequencing data read using the module for reading sequencing data for any one of the microsatellite loci i,

the module for determination for use in determining whether any one of the microsatellite loci *i* is a microsatellite marker locus, the module for determination comprising a first analysis module, a second analysis module, and a third analysis module,

the first analysis module is used to determine the length characteristics of the locus repetitive sequence under microsatellite stable (MSS), and determine whether the number of corresponding reads in the MSS sample is greater than 75% of the total number of reads supported by the locus, wherein length characteristics of MSS is a minimum range of continuous length, and it is recorded as "+" if a positive result is obtained and it is recorded as "-" if a negative result is obtained,

the second analysis module is used to determine the length characteristics of the locus repetitive sequence under microsatellite instability-high (MSI-H), wherein the length characteristics of MSI-H is a range of continuous length that is highly differentiated in MSS and MSI-H samples, and determine that a) whether the total number of reads supported within the range of continuous length is less than 0.2% of the total number of reads at the locus in the MSS sample, which is recorded as "+" if a positive result is obtained and recorded as "-" if a negative result is obtained,

and b) whether the reads account for more than 50% of the total number of reads at the locus in the MSI-H sample, which is recorded as "+" if a positive result is obtained and recorded as "-" if a negative result is obtained, the third analysis module is used to analyze the results of the first analysis module and the second analysis module, and determine the microsatellite locus I as a microsatellite marker locus if three positive results are obtained, i.e. three "+"s.

24. The device of claim 23, wherein the sample includes a sample from normal white blood cells and tissues from cancer patients, and the cancer is preferably colorectal cancer (such as bowel cancer), gastric cancer or endometrial cancer.

25. The microsatellite locus determined by the device of claim 23, comprising one or more of the 8 microsatellite loci described in Table 1.

26. The device according to claim 23, wherein the detection of microsatellite instability is used for non-invasive diagnosis, prognostic evaluation, selection of treatment or genetic screening of cancer, preferably colorectal cancer (such as bowel cancer), gastric cancer or endometrial cancer.

27. A device for determining the microsatellite instability of a plasma sample of a cancer patient based on the next-generation high-throughput sequencing method, **characterized in that** the device comprises:

the module for reading sequencing data for use in reading the sample sequencing data obtained and stored in the sequencing equipment,

the module for determining the length characteristics of repetitive sequences for use in analyzing the length characteristics of repetitive sequences of multiple microsatellite loci in a plasma sample and an MSS plasma sample as the reference sample from the sample sequencing data, the multiple microsatellite loci comprising one or more of microsatellite loci selected from the 8 microsatellite loci shown in Table 1;

the module for calculating enrichment index for use in calculating enrichment index Zscore for the microsatellite loci;

the module for calculating the microsatellite status index for use in summing the enrichment index Zscore of all microsatellite loci to result in the index MSscore for judging the microsatellite stability status of the sample;

the module for calculating the threshold for use in calculating the mean and standard deviation SD of the MSscore of the MSS plasma sample as the reference sample, with mean + 3SD as the threshold cutoff;

the template for determining the stability status of microsatellite loci for use in comparing index MSscore with threshold cutoff, and determining the sample as MSI-H when MSscore > cutoff and determining the sample as MSS when MSscore $\leq$ cutoff for a plasma sample from a cancer patient.

**28.** The device of claim 27, **characterized in that** the Zscore is evaluated by $H_s$, evaluated by $H_s = - \log(P_s(X > k_s))$, and

$$P(X = k) = \frac{\binom{K}{k}\binom{N-k}{n-k}}{\binom{N}{n}}$$

wherein N is the total number of reads in the repetitive sequence length set for MSI-H status and MSS status, K is the total number of reads in the repetitive sequence length set for MSI-H status, and N - K is the total number of reads in the repetitive sequence length set for MSS status, and correspondingly, $n$ and $k$ are the number of respective reads in the sample to be tested, respectively.

**29.** The device of claim 27, **characterized in that** MSscore is calculated based on the following formula:

$$MSscore = \sum_{s \in \text{markers}} \frac{H_s - \underset{\text{MSS\_Sample } s}{mean}(H_s)}{\underset{\text{MSS\_Sample } s}{sd}(H_s)}$$

**30.** The device of claim 27, **characterized in that** the disease is cancer, preferably colorectal cancer (such as bowel cancer), gastric cancer, or endometrial cancer.

(A)

(B)

FIG. 1

FIG. 2

## INTERNATIONAL SEARCH REPORT

| International application No. |
| --- |
| **PCT/CN2019/109036** |

**A. CLASSIFICATION OF SUBJECT MATTER**

C12Q 1/68(2018.01)i; C12N 15/11(2006.01)i; G16B 30/00(2019.01)i; G16H 50/30(2018.01)i

According to International Patent Classification (IPC) or to both national classification and IPC

**B. FIELDS SEARCHED**

Minimum documentation searched (classification system followed by classification symbols)

C12Q; C12N; G16B; G16H

Documentation searched other than minimum documentation to the extent that such documents are included in the fields searched

Electronic data base consulted during the international search (name of data base and, where practicable, search terms used)

CNABS; SIPOABS; DWPI; CNTXT; EPTXT; USTXT; WOTXT; CNKI; ISI WEB OF SCIENCE; ELSEVIER; 百度学术, BAIDU SCHOLAR; CPRSABS: AKT1, 血浆, 燃石医学, bMS-BR1, bMS-BR, 标志物, 微卫星不稳定, msi, biomarker, serum, 11号染色体, 102193508

**C. DOCUMENTS CONSIDERED TO BE RELEVANT**

| Category* | Citation of document, with indication, where appropriate, of the relevant passages | Relevant to claim No. |
| --- | --- | --- |
| PX | CN 109207594 A (GUANGZHOU BURNING ROCK MEDICAL EXAMINATION INSTITUTE CO., LTD.) 15 January 2019 (2019-01-15)<br>claims 1-19 | 12-30 (in part) |
| PX | CN 109182525 A (GUANGZHOU BURNING ROCK MEDICAL EXAMINATION INSTITUTE CO., LTD.) 11 January 2019 (2019-01-11)<br>claims 1-11 | 1-11 (in part) |
| X | CN 106755501 A (GUANGZHOU BURNING ROCK MEDICAL EXAMINATION INSTITUTE CO., LTD.) 31 May 2017 (2017-05-31)<br>claims 1-18, and description, paragraphs 22-26 and 33 | 12, 13, 15 (all in part) |
| X | CN 107513565 A (GENESEEQ TECHNOLOGY INC.) 26 December 2017 (2017-12-26)<br>claims 1-8, table 1, and description, paragraphs 61-78 | 1-11, 14, 20-30 (in part) |
| Y | CN 107513565 A (GENESEEQ TECHNOLOGY INC.) 26 December 2017 (2017-12-26)<br>claims 1-8, table 1, and description, paragraphs 61-78 | 16-19 (in part) |
| Y | CN 106755501 A (GUANGZHOU BURNING ROCK MEDICAL EXAMINATION INSTITUTE CO., LTD.) 31 May 2017 (2017-05-31)<br>claims 1-18, and description, paragraphs 22-26 and 33 | 16-19 (in part) |

☐ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **17 December 2019** | **03 January 2020** |

| Name and mailing address of the ISA/CN | Authorized officer |
| --- | --- |
| **China National Intellectual Property Administration**<br>**No. 6, Xitucheng Road, Jimenqiao Haidian District, Beijing**<br>**100088**<br>**China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2019/109036** |

**Box No. III    Observations where unity of invention is lacking (Continuation of item 3 of first sheet)**

This International Searching Authority found multiple inventions in this international application, as follows:

[1]    Invention 1: a combination of bMS-BR1 and AKT1;

[2]    Invention 2: a combination of bMS-BR1 and APC

[3]    ......

[4]    Invention 328: a combination of bMS-BR8 and UGT1A1;

[5]    The combinations of biomarkers are different from each other, do not share the same or corresponding special technical feature, and therefore lack unity of invention (PCT Rule 13).

1. ☐    As all required additional search fees were timely paid by the applicant, this international search report covers all searchable claims.

2. ☐    As all searchable claims could be searched without effort justifying additional fees, this Authority did not invite payment of additional fees.

3. ☐    As only some of the required additional search fees were timely paid by the applicant, this international search report covers only those claims for which fees were paid, specifically claims Nos.:

4. ☑    No required additional search fees were timely paid by the applicant. Consequently, this international search report is restricted to the invention first mentioned in the claims; it is covered by claims Nos.: **1-30 (the technical solution relating to the combination of bMS-BR1 and AKT1)**

**Remark on Protest**    ☐    The additional search fees were accompanied by the applicant's protest and, where applicable, the payment of a protest fee.

☐    The additional search fees were accompanied by the applicant's protest but the applicable protest fee was not paid within the time limit specified in the invitation.

☐    No protest accompanied the payment of additional search fees.

Form PCT/ISA/210 (continuation of first sheet) (January 2015)

**INTERNATIONAL SEARCH REPORT**
Information on patent family members

International application No.

**PCT/CN2019/109036**

| Patent document cited in search report | | | Publication date (day/month/year) | Patent family member(s) | | | Publication date (day/month/year) |
|---|---|---|---|---|---|---|---|
| CN | 109207594 | A | 15 January 2019 | None | | | |
| CN | 109182525 | A | 11 January 2019 | CN | 109182525 | B | 06 September 2019 |
| CN | 106755501 | A | 31 May 2017 | WO | 2018137678 | A1 | 02 August 2018 |
| CN | 107513565 | A | 26 December 2017 | CN | 107513565 | B | 24 August 2018 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 201811149011 A
- WO 201811149015 A

- CN 201710061152